# EUROPÄISCHE PATENTSCHRIFT

(11) **EP 2 725 962 B1**
(45) Veröffentlichungstag und Bekanntmachung des Hinweises auf die Patenterteilung: **29.07.2020**
(21) Anmeldenummer: 12735776.2
(22) Anmeldetag: 04.06.2012
(51) Int. Cl.: A61B 1/00, A61B 17/34

(54) **TROKARSYSTEM**
TROCAR SYSTEM
SYSTÈME DE TROCART

(30) Priorität: 30.06.2011 DE 102011107612
(43) Veröffentlichungstag der Anmeldung: 07.05.2014
(73) Patentinhaber: Riek, Siegfried, 78628 Rottweil (DE); Bachmann, Karl-Heinz, 78667 Villingendorf (DE); Gaiselmann, Thomas, 78667 Villingendorf (DE)
(72) Erfinder: Riek, Siegfried, 78628 Rottweil (DE); Bachmann, Karl-Heinz, 78667 Villingendorf (DE); Gaiselmann, Thomas, 78667 Villingendorf (DE)
(74) Vertreter: Westphal, Mussgnug & Partner Patentanwälte mbB
(86) Internationale Anmeldenummer: PCT/EP2012/002353
(87) Internationale Veröffentlichungsnummer: WO 2013/000536

(56) Entgegenhaltungen:
- EP-A1- 1 977 696
- EP-A2- 2 133 027
- DE-A1- 10 333 956
- US-A- 5 916 233
- US-A- 6 007 483
- US-A1- 2005 159 764

## Beschreibung

Die Erfindung betrifft einen Trokardorn gemäß dem Oberbegriff des Patentanspruchs 1. Weiterhin betrifft die Erfindung ein Trokarsystem nach Anspruch 8 mit einem derartigen Trokardorn.

Trokarsysteme für die minimal-invasive Chirurgie bestehen in der Regel aus einem Trokardorn, um eine Öffnung in eine Körperhöhle (z.B. Bauchraum) zu schaffen, und einer Trokarhülse, die in dieser Öffnung verbleibt und einen Zugang zu dem Inneren der Körperhöhle für den operativen Eingriff bildet. Der Trokardorn weist eine distale Spitze auf, die zur Penetration des Körpergewebes, z.B. der Baudecke, dient, um die Öffnung zu schaffen. Die Spitze des Trokardorns kann dabei scharf, schneidend oder stumpf ausgebildet sein. Eine scharfe Spitze hat beispielsweise die Form einer dreikantigen Pyramide. Schneidende Spitzen weisen eine Klinge auf, die eine Gewebeinzision schafft, welche anschließend durch die konische Spitze dilatiert wird. Stumpfe Spitzen sind distal abgerundet, so dass sehr hohe Penetrationsdrucke für die Eröffnung der Gewebeschichten notwendig sind. Danach bewirken diese im Wesentlichen nur eine Dilatation einer zuvor erzeugten Läsion.

Bei diesen Trokaren, insbesondere den spitzen und schneidenden Trokaren besteht die Gefahr, dass bei der Penetration der Bauchdecke durch Verwachsungen am Peritoneum anhaftende innere Organe, z.B. Darm bzw. Blutgefäße in der Bauchdecke oder im Retroperitoneum verletzt werden. Um dieses Risiko zu reduzieren werden sogenannte optische Trokare verwendet, wie sie beispielsweise in der US 5,685,820 A beschrieben sind. Bei diesen optischen Trokaren ist die distale Spitze als hohler durchsichtiger Kegel ausgebildet, der von innen durch eine Optik beobachtbar ist, welche in einem koaxial in dem Trokardorn verlaufenden Optikkanal aufgenommen ist. Der optische Trokar ermöglicht durch die durchsichtige Spitze eine dreidimensionale Sicht auf die Gewebeschichten der Bauchdecke, durch welche die Spitze des Trokars hindurchtritt. Dadurch bekommt der Operateur ein Gefühl für die Bewegung, die Geschwindigkeit und die Position der Trokarspitze bei der Penetration. Insbesondere kann vor der Penetration des Peritoneums erkannt werden, ob Adhäsionen zwischen Darm und Peritoneum an der Insertionsstelle vorhanden sind. Problematisch bleiben die hohen Penetrationsdrücke bei der Penetration der Fascie und des Peritoneums. Diese Penetrationsdrücke könnten zwar durch schneidende Klingen an der Trokarspitze reduziert werden, dies würde jedoch wiederum die Gefahr einer Verletzung des Darmes bei der Penetration erhöhen. Zur Reduktion des Penetrationsdruckes wird der bekannte optische Trokar mit oder ohne schabende Kufen als Kompromiss zwischen Druckminderung und Verletzungsrisiko verwendet, wobei auch in diesem Falle relativ hohe Penetrationsdrucke und permanent rotierende Bewegungen des Trokars erforderlich sind. Auch hierbei entsteht der sogenannte Tentingeffekt, bei welchem die Gewebeschichten, die einen hohen Penetrationsdruck erfordern, von der Trokarspitze zeltartig in das Abdomen gedrückt werden und sich dicht dem Retroperitoneum nähern können. Beim Eröffnen dieser Schichten geben diese dann plötzlich dem Penetrationsdruck nach und die Spitze dringt schlagartig in das Abdomen ein und der Operateur kann Schwierigkeiten haben, die Bewegung des Trokars zu kontrollieren, um nicht mit der Spitze des Trokars innere Organe oder große Gefäße des Retroperitoneums zu verletzen. Um dieses Problem zu reduzieren, wird von zahlreichen Operateuren die Minilaparotomie eingesetzt. Dabei wird über einen Hautschnitt die Bauchdecke in klassischer Weise mit dem Skalpell aufpräpariert und dann eine Trokarhülse in das eröffnete Peritoneum eingesetzt.

Aus der US 2005/159764 A1 ist ein Instrument zur Präparation einer Beinvene bekannt, die für eine Bypass-Operation verwendet wird. Das Instrument weist einen als Disektor dienenden Trokardorn auf, in welchem koaxial ein Optikkanal verläuft, durch welchen eine Optik eingeführt werden kann. Mittels der Optik kann die durchsichtige distale Spitze des Trokardorns beobachtet werden. In der Wandung des Trokardorns ist wenigstens ein Kanal ausgebildet, der als Insufflationskanal dient, durch welchen ein Insufflationsgas eingeleitet werden kann, welches durch eine distale Austrittsöffnung ausstritt. Ähnliche Instrumente sind aus EP 2 133 027 A2 und EP 1 977 996 A1 bekannt. Die Insufflationskanäle weisen sich stufenförmig ändernden Querschnitt auf und die Austrittsöffnungen münden gegen den Insufflationskanal abgewinkelt seitlich an der distalen Spitze. Die Insufflationskanäle eignen sich daher ausschließlich für das Einleiten eines Fluids, insbesondere eines Gases.

Aus US 5 916 233 A ist ein Disektor-Instrument zur Präparation einer Beinvene bekannt, welche einen hohlen Trokardorn aufweist, in welchem eine Optik einführbar ist, mittels derer die durchsichtige distale Spitze des Trokardorns von innen beobachtet werden kann. Der Optikkanal verläuft exzentrisch in dem Trokardorn. Ein Miniaturinstrument kann durch den Trokardorn hindurchgeführt werden und tritt distal neben der Spitze des Trokardorns aus.

Problematisch ist die Abdichtung des Pneumoperitoneums, da die entstandene Öffnung in der Bauchdecke größer ist als dies bei einem Trokareinstich der Fall wäre. Die offene Inzision der Bauchdecke widerspricht dabei allerdings der Zielsetzung der minimal-invasiven Operationstechnik.

Der Erfindung liegt die Aufgabe zu Grunde, ein Trokarsystem zur Verfügung zu stellen, welches die Vorteile des optischen Trokars ausnützt, ohne hohe Drücke bei der Gewebepenetration zu benötigen.

Diese Aufgabe wird erfindungsgemäß gelöst durch ein Trokarsystem mit den Merkmalen des Patentanspruchs 1.

Vorteilhafte Ausführungen der Erfindung sind in den Unteransprüchen angegeben.

Der wesentliche Gedanke der Erfindung besteht darin, den Trokar nicht nur als passives Werkzeug zu verwenden, welches manuell durch eine axiale Kraft und gegebenenfalls durch eine Rotationsbewegung durch die Gewebeschichten geführt wird. Dem Trokar können vielmehr erfindungsgemäß verschiedene aktive chirurgische Instrumente zugeordnet werden, die durch wenigstens einen Arbeitskanal, der in dem Trokardorn ausgebildet ist, hindurchgeführt und an der distalen Spitze des Trokars ausgefahren werden können. Dadurch ist es für den Operateur möglich, unmittelbar an der distalen Trokarspitze aktiv chirurgisch tätig zu werden, ohne dass zusätzlich zu dem Trokar ein weiterer Zugang geschaffen werden muss. Über die Optik und die durchsichtige distale Spitze des Trokardorns kann der Operateur die chirurgischen Vorgänge mittels der durch die Arbeitskanäle ausgefahrenen Instrumente unter Sicht durchführen. Es steht eine große Anzahl unterschiedlicher Instrumente in einer miniaturisierten Ausführung zur Verfügung, die sich für das Hindurchführen durch die Arbeitskanäle eignen. Eine entsprechende Vielzahl von unterschiedlichen Eingriffen wird durch das erfindungsgemäße Trokarsystem ermöglicht.

Für das Einführen des Trokars durch die verschiedenen Gewebeschichten, insbesondere der Bauchdecke, kann eine miniaturisierte Schere oder ein Messer aus der distalen Trokarspitze ausgefahren werden, um die jeweilige Gewebeschicht vor der distalen Spitze zu durchtrennen oder einzuschneiden. Insbesondere die widerstandsfähigen Gewebeschichten, wie die Fascie und das Peritonemum können auf diese Weise unter Sicht durch eine kleine Inzision geöffnet werden, sodass anschließend die Spitze des Trokardorns in diese Inzision eindringen kann und ohne stärkeren Druck und insbesondere ohne Tentingeffekt die Inzision dilatieren und die Gewebeschicht penetrieren kann. Ähnlich der Technik der offenen Laparotomie können somit die jeweils an der distalen Spitze des Trokars anliegenden visualisierten Gewebeschichten präpariert werden, um ein nahezu druckloses und damit risikoloses Penetrieren der Gewebeschichten zu ermöglichen. Die Semitransparenz des Peritroneums erlaubt bei der Penetration des optischen Trokars das Erkennen von Adhäsionen vor der Eröffnung. Dies ist bei der offenen Laparotomie nicht möglich.

Weiter können durch die Arbeitskanäle Pinzetten oder Fasszangen hindurchgeführt werden, um das Gewebe an der distalen Trokarspitze festzuhalten, was insbesondere für eine Inzision mittels einer durch einen weiteren Arbeitskanal eingeführten Schere oder Klinge vorteilhaft sein kann.

Bei dem ersten Einstich ist es weiter in vorteilhafter Weise möglich, durch einen Arbeitskanal eine miniaturisierte Veressnadel einzuführen. Mit dieser Veressnadel kann unter Sicht das Peritonemum durchstoßen werden, um dann über die Veressnadel den Bauchraum zu insufflieren.

Weiter können durch die Arbeitskanäle Klemmen oder Koagulationsinstrumente hindurchgeführt und an der distalen Spitze ausgefahren werden. Mit diesen Instrumenten können Gefäße abgeklemmt bzw. koaguliert werden.
Weiter können auch miniaturisierte Morcellatoren über die distale Spitze ausgefahren werden.

Weiter ist es auch möglich, einen miniaturisierte Digitalkamera durch einen Arbeitskanal einzuführen, um z.B. den Operationsvorgang aus einer anderen Perspektive zu dokumentieren. Es können auch Lichtleiter für eine zusätzliche Beleuchtung durch einen Arbeitskanal geführt werden.

Das erfindungsgemäße Trokarsystem ermöglicht somit die Penetration insbesondere der Bauchdecke, wozu beispielsweise der Trokar eingestochen wird, bis seine distale Spitze die Fascie erreicht. Über einen Arbeitskanal wird eine Klemme ausgefahren, die die Fascie hält, während durch einen anderen Arbeitskanal eine Schere ausgefahren wird, die die Fascie eröffnet. Dies geschieht unter Sicht durch die durchsichtige Spitze des Trokardorns. Die Spitze des Trokardorns wird nun in die erzeugte Öffnung der Fascie eingebracht, wobei die weitere Dilatation atraumatisch mit geringem Penetrationsdruck erfolgt. Erreicht die Trokarspitze das Peritoneum, so wird durch ein atemsynchrones Verschieben des Darmes festgestellt, dass keine Adhäsionen vorliegen, worauf in entsprechender Weise das Peritoneum mittels Schere und gegebenenfalls Klemme eröffnet werden kann. Die Spitze des Trokardorns wird dann in die erzeugte Öffnung des Peritoneums eingebracht. Auch hierbei ist kein nennenswerter Penetrationdruck erforderlich, sodass der Tentingeffekt mit seinen Risiken vermieden wird. Dieses Vorgehen ist analog zu den gewohnten präparativen Schritten bei der offenen Laparotomie. Zum Unterschied zu diesem bekannten präparativen Vorgehen ist jedoch kein größerer Schnitt notwendig, als für das Einbringen des Trokars erforderlich ist. Alternativ kann bei Erreichen des Periotoneums auch eine Veressnadel durch einen Arbeitskanal ausgefahren werden, mit welcher dann das Peritoneum unter Sicht penetriert wird, um dann CO₂ in das Abdomen zu insufflieren. Sobald durch die Insufflation das Peritoneum von den Därmen distanziert ist, wird die Veressnadel zurückgezogen und die Spitze des Trokars wird in die durch die Veressnadel gebildete Öffnung eingebracht, um diese unter geringem Druck atraumatisch zu dilatieren.

Bei dem erfindungsgemäßen Trokarsystem kann mittels des Trokardorns eine Trokarhülse eingesetzt werden, die dann als Zugangskanal für den nachfolgenden minimal-invasiven Eingriff dient. Ebenso ist es möglich, eine Single-Port-Operation mittels des Trokarsystems durchzuführen, ohne eine Trokarhülse zu setzen. In diesem Falle verbleibt der Trokardorn mit der Optik und den Arbeitskanälen als einziger Zugang für den anschließenden minimal-invasiven Eingriff, wobei die Instrumente für den Eingriff durch die Arbeitskanäle eingeführt werden.

Die mit dem erfindungsgemäßen Trokarsystem verwendbaren Instrumente sind im Wesentlichen an sich bekannte chirurgische Miniaturinstrumente. Diese weisen ein an der distalen Spitze des Trokardorns ausfahrbares Arbeitselement auf, während an dem proximal außerhalb des Arbeitskanals verbleibenden Ende die proximalen Betätigungselemente des Miniaturinstruments angeordnet sind. Die Instrumente können mit einem starren oder einem flexiblen Schaft ausgebildet sein. Bei flexiblen Instrumenten ist es möglich, diese so elastisch vorzuspannen, dass sich ihre distalen Arbeitselemente beim Austritt aus der distalen Spitze des Trokars gegen die Mittelachse des Trokars biegen, um ein präparatives Arbeiten unmittelbar vor der durchsichtigen Spitze zu ermöglichen.

Alternativ kann in dem Arbeitskanal auch ein Führungsröhrchen axial verschiebbar und drehbar angeordnet sein, durch welches das Miniaturinstrument durchgeführt wird. Das Führungsröhrchen ist in seinem distalen Endbereich elastisch auf Biegung vorgespannt. Vorzugsweise besteht das Führungsröhrchen aus einer Memory-Legierung mit superelastischen Eigenschaften, z. B. aus Nitinol. Wird das Führungsröhrchen distal aus dem Arbeitskanal herausgeschoben, so krümmt sich sein distales Ende aus seiner durch den Arbeitskanal festgelegten Längsachse, wobei der Ablenkwinkel gegenüber der Längsachse zunimmt, je weiter das distale Ende des Führungsröhrchens aus dem Arbeitskanal austritt. Durch Drehen des Führungsröhrchens in dem Arbeitskanal kann dabei die Richtung der Ablenkung um die Längsachse gedreht werden. Durch axiales Verschieben und Drehen des Führungsröhrchens kann somit die Austrittsrichtung und Positionierung des distalen Arbeitselements des Miniaturinstruments dreidimensional gesteuert werden. Am proximalen Ende vorgesehene Stellmittel erlauben die axiale und rotatorische Bewegung des Führungsröhrchens in dem Arbeitskanal.

Um ein Entweichen des Gases aus dem insufflierten Bauchraum zu verhindern, können die Arbeitskanäle proximal durch ein Ventil verschließbar sein, wenn sich kein Instrument in dem Arbeitskanal befindet. Ein solches Ventil kann insbesondere durch eine an sich bekannte Lippendichtung gebildet sein, die ein Durchtreten eines Instruments erlaubt und ein eingesetztes Instrument an dessen Außenumfang abdichtet.

Weiter können unbenutzte Arbeitskanäle durch einen Mandrin verschlossen werden, der die distale Austrittsöffnung des Arbeitskanals verschließt, sodass insbesondere eine Verunreinigung des Arbeitskanals vermieden wird.

Herstellungstechnisch kann der Trokardorn eine den koaxialen Optikkanal umschließende massive Wandung aufweisen, in welcher die Arbeitskanäle als achsparallel durchgehende Bohrungen ausgebildet sind. Alternativ ist es möglich, den Trokardorn mit einer doppelten Wandung auszubilden, die einen hohlen Ringspalt bildet. In diesem Ringspalt sind die Arbeitskanäle als achsparallel durchgehende Röhrchen angeordnet. Bei ausreichenden radialen Platzverhältnissen können die Arbeitskanäle auch als Röhrchen auf der inneren oder äußeren Mantelfläche der Wandung angebracht sein bzw. bei einem Trokardorn aus Kunststoff eingegossen sein. Die Zahl der Arbeitskanäle wird durch den Verwendungszweck bestimmt. Im einfachsten Falle ist nur ein Arbeitskanal vorhanden. Vorzugsweise sind jedoch zwei oder drei Arbeitskanäle im gleichen gegenseitigen Winkelabstand vorgesehen. Die distalen Austrittsöffnungen der Arbeitskanäle können in gleicher axialer Position in der Mantelfläche der distalen Spitze des Trokardorns angeordnet sein. Ebenso ist es möglich, dass die Arbeitskanäle distal in unterschiedlicher axialer Lage an der Spitze des Trokardorns münden. Die Arbeitskanäle verlaufen im Wesentlichen achsparallel in dem Trokardorn oder auch in einem Winkel zur Intrumentenachse oder schraubenförmig um die Instrumentenachse. Am proximalen Ende können sie unter einem Winkel zur Trokarachse eintreten und/oder am distalen Ende unter einem Winkel zur Trokarachse austreten. Ein abgewinkeltes Eintrittsende kann gegebenenfalls das Einführen und Handhaben des Miniaturinstruments erleichtern. Ein abgewinkeltes Austrittsende kann die Positionierung des Arbeitselements des Miniaturinstruments erleichtern, insbesondere wenn dieses elastisch auf Biegung vorgespannt ist. Die Eintrittsöffnung und die Austrittsöffnung des Arbeitskanals müssen nicht notwendig am proximalen bzw. distalen Ende des Trokardorns angeordnet sein, sondern können auch axial gegen die Enden des Trokardorns versetzt angeordnet sein.

Ein neues Einsatzgebiet kann das erfindungsgemäße Trokarsystem in der Mammachirurgie finden. Tumore oder auch Lymphknoten können unter Sicht z.B. von einem kosmetisch günstigen Mamillenrandschnitt angefahren werden. Am Ort des Tumors können dann sämtliche erforderlichen chirurgischen Instrumente über die Arbeitskanäle zum Einsatz kommen. Diese Single-Port-Technik, bei welcher der Trokardorn auch zur Zuführung der Instrumente für den eigentlichen chirurgischen Einsatz dient, ist dabei insbesondere unter kosmetischem Gesichtspunkt von Vorteil, da nur ein einziger Einschnitt erforderlich ist. Weiter kann über einen Arbeitskanal eine CO₂-Insufflation durchgeführt werden, um einen erweiterten Raum zu erzeugen, so dass die präparative Operation in einer nicht präformierten Gewebshöhle stattfinden kann. Der gesamte Eingriff kann dabei unter Sicht durch die distale Spitze des Trokardorns erfolgen, sodass eine exakte und vollständige Präparation und Entfernung eines Tumors möglich ist. Es können alle bekannten Operationsinstrumente und Operationstechniken zum Einsatz kommen, z.B. Messer, Stanzen, Koagulationsinstrumente, Morcellatoren, Optiken, Lichtleiter, Beleuchtungssysteme und dergleichen.

Im Folgenden wird die Erfindung anhand eines in der Zeichnung dargestellten Ausführungsbeispiels näher erläutert. Es zeigen:
- Fig. 1: einen Axialschnitt durch ein Trokarsystem, und
- Fig. 2: einen Schnitt gemäß der Linie A-A in Figur 1.

Das Trokarsystem weist einen Trokardorn 10 auf. Der Trokardorn 10 hat die Form eines starren langgestreckten zylindrischen Rohres, welches aus Metall oder Kunststoff gefertigt ist. Das Innenlumen des Trokardorns 10 bildet einen koaxial vom proximalen Ende zum distalen Ende führenden Optikkanal 12. Die distale Spitze 14 des Trokardorns 10 hat die Form eines Kegels mit abgerundet stumpfer Spitze. Der Kegelmantel kann gegebenenfalls auch ballig ausgewölbt sein. Die kegelförmige Spitze 14 ist im Inneren hohl und besteht aus einem dünnwandigen durchsichtigen Material, insbesondere aus einem durchsichtigen Kunststoff. In den Optikkanal 12 des Trokardorns ist eine Optik 16 einsetzbar, die insbesondere als Stablinsenoptik oder mit einem Kamerachip ausgebildet sein kann und ein Beleuchtungssystem integriert haben kann. Bei eingesetzter Optik 16 befindet sich deren distales Ende etwa im Bereich der Basisfläche der kegelförmigen Spitze 14. Durch die Optik 16 kann die distale Spitze 14 von innen ausgeleuchtet und beobachtet werden. Dadurch ist es möglich, dass an der Außenseite der distalen Spitze 14 anliegende Gewebe zu beobachten.

Insoweit entspricht das Trokarsystem einem an sich bekannten optischen Trokar. Abwandlungen, wie sie von optischen Trokaren bekannt sind, sind ebenfalls möglich und erfindungsgemäß verwendbar.

In der den Optikkanal 12 umschließenden Wandung 18 des Trokardorns 10 ist wenigstens ein Arbeitskanal 20 vorgesehen. Im dargestellten Ausführungsbeispiel sind zwei diametral zueinander angeordnete Arbeitskanäle 20 vorgesehen. Andere Ausführungen mit drei oder auch mehr Arbeitskanälen 20 sind möglich, die dann vorzugsweise jeweils im gleichen Winkelabstand gegeneinander versetzt um den Optikkanal 12 herum angeordnet sind.

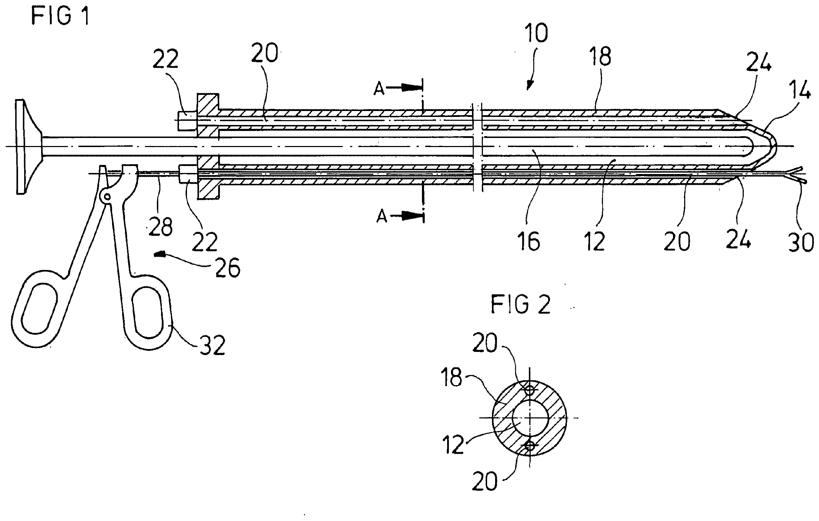

Die Arbeitskanäle 20 verlaufen in der Wandung 18 des Trokardorns 10 durchgehend von proximal nach distal. Im Ausführungsbeispiel der Figuren 1 und 2 sind die Arbeitskanäle 20 als Bohrungen in der massiven Wandung 18 ausgebildet. Alternativ kann die Wandung 18 auch durch zwei koaxiale hülsenartige Rohre gebildet werden, in deren kreiszylindrischem freien Zwischenraum die Arbeitskanäle 20 als dünne Röhrchen angeordnet sind. Der Trokardorn 10 kann insbesondere auch aus Kunststoff hergestellt sein. In diesem Falle können die Arbeitskanäle 20 beim Gießen des Kunststoffs erzeugt werden oder als Röhrchen mit dem Kunststoff umspritzt werden. Besteht der Trokardorn aus einem durchsichtigen Kunststoff, so können die Arbeitskanäle 20 und ein in den Arbeitskanal 20 eingeführtes Miniaturinstrument von außen beobachtet werden.

In einer Ausführung für Operationen im Bauchraum kann beispielsweise der Trokardorn 10 einen Außendurchmesser von 14 mm, einen Durchmesser des Optikkanals 12 von 5 mm und einen Durchmesser der Arbeitskanäle 20 von 3 mm aufweisen.

Am proximalen Ende sind die Arbeitskanäle 20 vorzugsweise durch ein Ventil 22 abgedichtet, welches das proximale Ende des jeweiligen Arbeitskanals 20 gasdicht verschließt, wenn der Arbeitskanal 20 leer ist, und welches ein abgedichtetes Hindurchführen eines Instruments erlaubt. Ein solches Ventil 22 kann in an sich bekannter Weise ausgebildet sein, z.B. als Lippendichtung oder dergleichen.

Die Arbeitskanäle 20 verlaufen im Allgemeinen achsparallel in der Wandung 18 des Trokardorns 10. Am proximalen Ende können die Arbeitskanäle 20 gegebenenfalls auch nach außen gegen die Mittelachse des Trokardorns 10 abgewinkelt sein. Ebenso können die Arbeitskanäle 20 an ihrem distalen Ende gegen die Achse des Trokardorns 10 nach außen abgewinkelt sein. Am distalen Ende münden die Arbeitskanäle 20 mit einer offenen Austrittsöffnung 24 im Bereich der distalen Spitze 14. Die Austrittsöffnungen 24 können sich dabei in gleicher axialer Position bezüglich der Längserstreckung des Trokardorns 10 befinden, wie dies in Figur 1 gezeigt ist. Es ist jedoch auch möglich, dass sich die Austrittöffnungen 24 der verschiedenen Arbeitskanäle 20 in ihrer axialen Position gegeneinander versetzt befinden, sodass die Austrittsöffnung 24 eines Arbeitskanals 20 weiter distal an der Spitze 14 mündet als die andere Austrittsöffnung 24, die beispielsweise auch proximal hinter der Spitze 14 liegen kann.

Durch die Arbeitskanäle 20 können jeweils Miniaturinstrumente 26 eingeführt werden, wie dies in Figur 1 für den unteren Arbeitskanal 20 beispielsweise gezeigt ist. Die Miniaturinstrumente 26 sind beliebige, dem jeweiligen Anwendungsfall entsprechende Instrumente, wie sie an sich bekannt sind. Die Miniaturinstrumente 26 weisen einen lang gestreckten Schaft 28 auf, an dessen distalem Ende jeweils ein Arbeitselement 30 angeordnet ist, welches mittels eines am proximalen Ende des Schaftes 28 angeordneten Betätigungselements 32 betätigt werden kann. In der Zeichnung ist nur als Beispiel eine Miniaturschere gezeigt, deren Arbeitselement 30 als Schere ausgebildet ist, während ihr Betätigungselement 32 ein Scherengriff ist.

Das Miniaturinstrument 26 wird von dem proximalen Ende her in den Arbeitskanal 20 eingeführt, wobei es durch das Ventil 22 hindurchtritt und durch dieses am Umfang abgedichtet wird. Das Miniaturinstrument 26 kann in dem Arbeitskanal 20 soweit vorgeschoben werden, bis das distale Arbeitselement 30 durch die Austrittsöffnung 24 ausgefahren ist und vor der distalen Spitze 14 zum Einsatz kommen kann. Miniaturinstrumente 26 sind in starrer, flexibler und semiflexibler Ausführung bekannt. Die Miniaturinstrumente 26 können insbesondere auch mit einer elastisch vorgespannten Schaftkrümmung ausgebildet sein. Dadurch weicht das distale Arbeitselement 30 aus der Achsrichtung des Arbeitskanals 20 ab, wenn das distale Arbeitselement aus der Austrittsöffnung 24 austritt. Dies ermöglicht ein gezieltes Positionieren des Arbeitselements 30 beim Einsatz.

In einer vorteilhaften Ausführung kann in dem Arbeitskanal 20 ein Führungsröhrchen eingesetzt sein, welches in dem Arbeitskanal axial bewegt und gedreht werden kann. Das Führungsröhrchen besteht aus einer Memory-Legierung mit sogenannten superelastischen Eigenschaften, z. B. aus Nitinol. Das Führungsröhrchen ist zumindest in seinem distalen Endbereich auf Krümmung vorgespannt. Wird das Führungsröhrchen in dem Arbeitskanal 20 distal vorgeschoben, so dass das distale Ende des Führungsröhrchens aus der Austrittsöffnung 24 austritt, so krümmt sich das distale Ende des Führungsröhrchens aus der Achsrichtung des Arbeitskanals, wobei die Ablenkung aus der Achsrichtung mit zunehmender Länge des austretenden Ende des Führungsröhrchens zunimmt. Durch Drehung des Führungsröhrchens in dem Arbeitskanal 20 kann zudem die Richtung des distalen Endes des Führungsröhrchens um die Achse gedreht werden. Wird das Miniaturinstrument 26 durch das Führungsröhrchen hindurch geführt, so kann die Austrittsrichtung des Arbeitselements 30 durch axiales Verschieben und Drehen des Führungsröhrchens dreidimensional gesteuert werden. Am proximalen Ende des Trokarsystems sind Stellmittel vorgesehen, die das axiale und rotatorische Bewegen des Führungsröhrchens ermöglichen.

Bei Bedarf kann in einen nicht genutzten Arbeitskanal 20 ein - in der Zeichnung nicht dargestellter - Mandrin eingesetzt werden, der mit seinem distalen Ende die Austrittsöffnung 24 flächenbündig verschließt, sodass ein Eindringen von Verunreinigungen in den nicht genutzten Arbeitskanal 20 verhindert wird.

Das Trokarsystem kann außer dem Trokardorn 10 noch eine auf diesen Trokardorn 10 aufgeschobene Trokarhülse aufweisen. Gewebeschichten, z.B. der Bauchdecke werden mittels des Trokardorns 10 penetriert, wobei die auf dem Trokardorn 10 sitzende Trokarhülse in die erzeugte Körperöffnung eingeführt wird. Nach dem Herausziehen des Trokardorns 10 verbleibt die Trokarhülse als Zugang zur Körperhöhle.

Alternativ kann der Trokardorn 10 auch ohne Trokarhülse als Single-Port verwendet werden. Nach der Penetration verbleibt der Trokardorn 10 in der erzeugten Körperöffnung und die Operation in der Körperhöhle wird unter Sicht durch die durchsichtige distale Spitze 14 mittels Miniaturinstrumenten durchgeführt, die durch die Arbeitskanäle 20 eingeführt werden.

### Bezugszeichenliste

- 10: Trokardorn
- 12: Optikkanal
- 14: distale Spitze
- 16: Optik
- 18: Wandung
- 20: Arbeitskanäle
- 22: Ventil
- 24: Austrittsöffnung
- 26: Miniaturinstrument
- 28: Schaft
- 30: Arbeitselement
- 32: Betätigungselement

## Patentansprüche

1. Trokardorn (10) mit einem in dem Trokardorn (10) verlaufenden Optikkanal (12) zur Aufnahme einer Optik (16), mit einer hohlen durchsichtigen distalen Spitze (14) des Trokardorns (16), die mittels der Optik (16) von innen beobachtbar ist, und mit wenigstens einem Kanal, in der den Optikkanal (12) umschließenden Wandung (18) des Trokardorns (10), der distal in einer Austrittsöffnung (24) mündet, wobei der wenigstens eine Kanal ein Arbeitskanal (20) ist, der durchgehend von proximal nach distal führt und durch den wenigstens ein Miniaturinstrument (26) in der Weise hindurchführbar ist, dass ein distales Arbeitselement (30) des Miniaturinstruments (26) distal aus der Austrittsöffnung (24) des Arbeitskanals (20) austritt und ein proximales Betätigungselement (32) des Miniaturinstruments (26) sich proximal außerhalb des Arbeitskanals (20) befindet,
**dadurch gekennzeichnet, dass** der Optikkanal (12) koaxial zur Außenmantelfläche der Wandung (18) und zur distalen Spitze (14) des Trokardorns (10) verläuft.

2. Trokardorn nach Anspruch 1
**dadurch gekennzeichnet, dass** die Spitze (14) die Form eines in distaler Richtung ausgewölbten stumpf abgerundeten Kegels aufweist.

3. Trokardorn nach Anspruch 2,
**dadurch gekennzeichnet, dass** die Austrittsöffnung (24) des wenigstens einen Arbeitskanals (20) im Bereich der distalen Spitze (14) liegt.

4. Trokardorn nach einem der vorliegenden Ansprüche,
**dadurch gekennzeichnet, dass** wenigstens zwei im Umfangswinkel gegeneinander versetzte Arbeitskanäle (20) vorgesehen sind, deren Austrittsöffnungen (24) in gleicher axialer Position oder axial gegeneinander versetzt angeordnet sind.

5. Trokardorn nach einem der Ansprüche 1 bis 4,
**dadurch gekennzeichnet, dass** der wenigstens eine Arbeitskanal (20) durch eine axial durchgehende Bohrung in der massiven Wandung (18) des Trokardorns (10) gebildet ist.

6. Trokardorn nach einem der Ansprüche 1 bis 4,
**dadurch gekennzeichnet, dass** der wenigstens eine Arbeitskanal (20) als Röhrchen ausgebildet ist, welches in dem freien Zwischenraum der als Doppelmantel ausgebildeten Wandung (18) des Trokardorns (10) angeordnet ist.

7. Trokardorn nach einem der Ansprüche 1 bis 4,
**dadurch gekennzeichnet, dass** der wenigstens eine Arbeitskanal (20) proximal durch ein Ventil (22) abgeschlossen ist.

8. Trokarsystem mit einem Trokardorn (10) nach einem der Ansprüche 1 bis 7 ,
**dadurch gekennzeichnet, dass** das Trokarsystem ein Miniaturinstrument (26) beinhaltet, welches eine Schere, ein Messer, eine Pinzette, eine Klemme, ein Koagulationsinstrument, eine Veressnadel, eine Digitalkamera, eine Optik, ein Lichtleiter oder ein Beleuchtungssystem ist.

9. Trokarsystem nach Anspruch 8,
**dadurch gekennzeichnet, dass** in dem wenigstens einen Arbeitskanal (20) ein Führungsröhrchen axial verschiebbar und drehbar angeordnet ist, durch welches das Miniaturinstrument (26) hindurchführbar ist, und dass das Führungsröhrchen zumindest in seinem distalen Endbereich auf Krümmung elastisch vorgespannt ist.

10. Trokarsystem nach Anspruch 9,
**dadurch gekennzeichnet, dass** das Führungsröhrchen aus einem Memory-Werkstoff mit superelastischen Eigenschaften, z. B. Nitinol besteht.

11. Trokarsystem nach einem der Ansprüche 8 bis 10,
**dadurch gekennzeichnet, dass** ein Mandrin in den wenigstens einen Arbeitskanal (20) einsetzbar ist und in eingesetztem Zustand die Austrittsöffnung (24) des Arbeitskanals (20) verschließt.

## Claims

1. Trocar obturator (10) with an optical cannula (12) extending through the trocar obturator (10) for receiving an optical device (16) having a hollow transparent distal tip (14) of the trocar obturator (10) that is observable from the inside by means of the optical device (16) and which comprises at least one cannula within a walling (18) that encloses the optical cannula (12) of the trocar obturator (10) which ends distally in an exit opening (24), wherein the at least one cannula is an operating cannula (20) extending continuously from proximal to distal and through which at least one miniature surgical instrument (26) can be introduced such that a distal operating element (30) of the miniature surgical instrument (26) exits the exit opening (24) of the operating cannula (20) and a proximal actuating element (32) of the miniature surgical instrument (26) is disposed proximally outside of the operating cannula (20), **characterized in that** the optical cannula (12) extends coaxially to the outer circumferential surface of the walling (18) and to the distal tip (14) of the trocar obturator (10).

2. Trocar obturator in accordance with claim 1,
**characterized in that** the tip (14) has a conical shape that is curved outwards in the distal direction and is blunt rounded.

3. Trocar obturator in accordance with claim 2,
**characterized in that** the exit opening (24) of the at least one operating cannula (20) is disposed in the region of the distal tip (14).

4. Trocar obturator in accordance with any of the preceding claims,
**characterized in that** at least two operating cannulae (20) are provided that are disposed offset to each other by the circumferential angle and of which the exit openings (24) are arranged in the same axial position or axially offset to each other.

5. Trocar obturator in accordance with any of claims 1 to 4,
**characterized in that** the at least one operating cannula (20) is formed by a continuous axial through hole in the solid walling (18) of the trocar obturator (10).

6. Trocar obturator in accordance with any of claims 1 to 4,
**characterized in that** the at least one operating cannula (20) is implemented as a small tube disposed within the vacant interspace of the walling (18), implemented as a double casing, of the trocar obturator (10).

7. Trocar obturator in accordance with any of claims 1 to 4,
**characterized in that** the at least one operating cannula (20) is closed off proximally by a valve (22).

8. Trocar system with a trocar obturator (10) in accordance with any of claims 1 to 7,
**characterized in that** the trocar system contains a miniature surgical instrument (26) comprising a scissors, a scalpel, a forceps, a clamp, a coagulation instrument, a Veress needle, a digital camera, an optical device, a light guide or an illumination system.

9. Trocar system in accordance with claim 8,
**characterized in that** a small guide tube is disposed axially moveable and rotatable within the at least one operating cannula (20) through which the miniature surgical instrument (26) can be introduced, and **in that** the small guide tube is elastically biased on curvature at least in the region of its distal end region.

10. Trocar system in accordance with claim 9,
**characterized in that** the small guide tube is comprised of a memory material with super-elastic characteristics, e.g. Nitinol.

11. Trocar system in accordance with any of claims 8 to 10,
**characterized in that** a mandrin can be inserted into the at least one operating cannula (20) which, when in an inserted state, closes off the exit opening (24) of the operating cannula.

## Revendications

1. Trocart (10) comportant un canal optique (12) traversant le trocart (10) pour recevoir une optique (16), une pointe distale creuse (14) transparente du trocart (10), qui s'observe de l'intérieur avec l'optique (16) et au moins un canal dans la paroi (18) du trocart (10) qui entoure le canal optique (12), débouchant en position distale dans un orifice de sortie (24),
- au moins un canal est un canal de travail (20) qui passe en continu de l'extrémité proximale jusqu'à l'extrémité distale et à travers lequel peut passer au moins un instrument miniature (26) de façon qu'un outil distal (30) de l'instrument miniature (26) sorte en position distale de l'orifice de sortie (24) du canal de travail (20) et un élément d'actionnement (32) proximal de l'instrument miniature (26) se trouve en position proximale à l'extérieur du canal de travail (20),
trocart **caractérisé en ce que**
le canal optique (12) passe coaxialement à la surface enveloppe de la paroi (18) et vers la pointe distale (14) du trocart (10).

2. Trocart selon la revendication 1,
**caractérisé en ce que**
la pointe (14) a la forme d'un cône arrondi bombé dans la direction distale.

3. Trocart selon la revendication 2,
**caractérisé en ce que**
l'orifice de sortie (24) d'au moins un canal de travail (20) se situe dans la zone de la pointe distale (14).

4. Trocart selon l'une des revendications précédentes,
**caractérisé en ce qu'**il comporte
au moins deux canaux de travail (20) décalés l'un par rapport à l'autre selon l'angle périphérique, dont les orifices de sortie (24) se situent dans la même position axiale ou dans des positions axialement opposées.

5. Trocart selon l'une des revendications 1 à 4,
**caractérisé en ce qu'**
au moins un canal de travail (20) est formé dans la paroi massive (18) du trocart (10) par un perçage axial traversant.

6. Trocart selon l'une des revendications 1 à 4,
**caractérisé en ce qu'**
au moins un canal (20) est réalisé sous la forme d'un petit tube est situé dans l'intervalle libre de la paroi (18) en forme de double enveloppe du trocart (10).

7. Trocart selon l'une des revendications 1 à 4,
**caractérisé en ce qu'**
au moins un canal de travail (20) est fermé à l'extrémité proximale par une soupape (22).

8. Système de trocart comportant un trocart (10) selon l'une des revendications 1 à 7,
**caractérisé en ce que**
le système de trocart comprend un instrument miniature (26) qui est une paire de ciseaux, un couteau, une pincette, une pince, un instrument de coagulation, une aiguille de Veress, une caméra numérique, une optique, un guide de lumière ou un système d'éclairage.

9. Système de trocart selon la revendication 8,
**caractérisé en ce qu'**
au moins un canal de travail (20) reçoit un petit tube de guidage coulissant axialement et un tournant, qui permet le passage de l'instrument miniature (26), le petit tube de guidage étant précontraint élastiquement en courbure au moins dans sa zone d'extrémité distale.

10. Système de trocart selon la revendication 9,
**caractérisé en ce que**
le petit tube de guidage est en un matériau à mémoire de forme ayant des caractéristiques super élastiques, par exemple, du nitinol.

11. Système de trocart selon l'une des revendications 8 à 10,
**caractérisé en ce qu'**
un mandrin peut se placer dans au moins un canal de travail (20) et, à l'état installé, il ferme l'orifice de sortie (24) du canal de travail (20).
